# EUROPEAN PATENT APPLICATION

(11) **EP 2 990 174 A1**
(43) Date of publication of application: **02.03.2016**
(21) Application number: 15181516.4
(22) Date of filing: 19.08.2015
(51) Int. Cl.: B29C 33/30, B29C 41/08, B29C 41/14, B29C 41/36, B29C 41/40

(54) **AUTOMATED PROSTHESIS SHELL SYSTEM AND METHOD**

(30) Priority: 21.08.2014 US 201414465424
(71) Applicant: Applied Silicone Company LLC, Santa Paula, CA 93060 (US)
(72) Inventor: WINN, R. Alastair, Santa Barbara, CA 93105 (US); PASKO, Nolan, Encinitas, CA 92024 (US)
(74) Representative: Hughes, Andrea Michelle

(57) **Abstract**

A system and method for automated manufacture of prosthesis shells using removably mountable mandrel is described. The mandrels are mounted onto cassettes 110, which are then moved through various dipping, devolatilization and curing steps by a robot 120, improving the uniformity of the shells and reducing cost of manufacture. The cassettes may include a motor 50 to rotatably drive spindles upon which the mandrels are mounted on the cassette. The motor may be driven by a battery housed within the cassette to provide for untethered use of the cassette.

## Description

The present invention relates to systems and methods for molding shells for fluid-filled prosthetic implants and, more particularly, to methods for rotationally molding breast prostheses shells.

### BACKGROUND

Implantable prostheses are commonly used to replace or augment body tissue. One such implantable prostheses are prostheses designed to augment or replace the human breast. Such implantable prostheses typically include a relatively thin and flexible envelope or shell made of vulcanized (cured) silicone elastomer. The shell is filled either with a silicone gel or with a normal saline solution. The filling of the shell may take place before or after the shell is implanted into a patient.

Traditional molding of implantable breast implant shells involves covering a mold or mandrel in uncured silicone dispersion by dipping the mold or mandrel into baths or by passing through a spray of silicone dispersion and allowing the dispersion to flow over the mandrel utilizing gravimetric forces. The shell may also be formed using rotational molding techniques. Whereas silicone, which is a term used to represent a family of materials formed from polysiloxanes, polymers in which the main chain consists of alternating silicon and oxygen atoms with organic side groups, is the most common material of construction, other materials such as polyurethane may be used.

In dip-molding, a suitably shaped mandrel is "dipped" into a dispersion of silicone elastomer and a solvent. The mandrel is withdrawn from the dispersion and the excess dispersion is allowed to drain from the mandrel. During and after the excess dispersion drains from the mandrel at least a portion of the solvent is allowed to evaporate to stabilize the silicone elastomer coating. The process is then repeated several times until a shell of the desired thickness is formed.

Once the shell is formed, the shell is removed from the mandrel. Typically, a patch is applied over the hole in the shell that remains after the mandrel has been removed. After the patch has been cured, if necessary, the hollow interior of the shell is filled with an appropriate gel, such as via a needle hole or valve formed in the patch. The needle hole or valve is then sealed, and the prosthesis may be further cured to complete any cross-linking of the gel or shell that is desired.

As mentioned previously, the shell may also be formed using a spray technique. Using such a technique can result in shells that have a non-uniform thickness. The shells may also be formed using rotational molding techniques. However such techniques require complicated processing techniques that may be more expensive than traditional techniques.

One common factor in all of the previous techniques is that they have been difficult and expensive to automate. Thus, in most cases, it is necessary to use manual labor to perform many of the tasks in manufacture of the shells.

Another problem with typical prosthesis shell manufacturing systems is that they require large clean room areas utilizing large single pass air handling systems to protect personnel operating within the clean room from solvent vapors given off by the manufacturing process. Such systems are costly to build, and costly to operate, and may also require costly systems to scrub the solvent vapors from the air once it has been evacuated from the clean room area to prevent contamination of the environment.

What has been needed, and heretofore unavailable is an automated system and method of manufacturing prostheses shells. Such a system and method would provide the advantage of consistent and uniform shell manufacture while reducing the need for manual labor to move mandrels and shells between various stations during forming of the shells. Such a system and method would also provide for more reliable manufacture with less waste of material and will allow a closed system for solvent vapor control, reducing the manufacturing clean room area required and provide improved solvent vapor emission control. The present invention satisfies these and other needs.

### SUMMARY OF THE INVENTION

In its most general aspect, the present invention includes a system and method employing mandrels that are configured to engage drive spindles that are rotatably driven by a cordless battery powered drive. The cassette may be coupled to an articulated arm using coupling devices so that the position of the cassette is easily manipulated by an operator or a computer controlled robot. The robot includes at least one articulated arm that can move in six axes, with the distal end of the articulated arm including a coupler that is configured to engage the cassette. In an alternative aspect, the articulated arm may include a drive motor or linkage associated with the coupler that drives the spindles of the cassette in a manner that results in rotation of the mandrels. In still another alternative aspect, the motor of the cassette may be powered using electrical power provided to the cassette through the coupler of articulated arm of the robot.

In another general aspect, use of the cassette and robot provides for an automated process for manufacturing prosthesis shells. In one aspect, the robot picks up a loaded cassette and positions the cassette under one or more nozzles from which a polymer dispersion, such as a silicone polymer dispersion, is pumped to apply a layer of polymer dispersion onto the one or more mandrels mounted on a cassette. Once the layer of polymer dispersion is laid down, the robot is controlled to place the cassette in a devolatilization station for a period of time to evaporate at least a portion of the solvent from the polymer dispersion. The process may be repeated to form additional layers, or alternatively, a barrier layer may be laid down over one or more base layers, and then devolatilized. The robot may then place the cassette into a curing oven to cure the base layers, and if present, the barrier layer, to complete the manufacture of the prosthesis shell.

In another aspect, the entire process, other than loading and unloading the mandrels from the cassette, may be automated using a centralized control system. In this aspect, a controller which includes a processor and a memory, with the processor programmed by appropriate programming commands stored or embedded in the memory, is programmed to control the operation of the manufacturing process, including operation of the robot, dispersion pumps, devolatilization station and curing oven.

In another aspect, the present invention includes a mounting system for mounting a mandrel configured to form a prosthesis shell to a cassette, comprising a cassette having a spindle, the spindle rotatably mounted to the cassette, the spindle having a proximal end configured to be driven so as to impart rotation to the spindle, the spindle also having a distal end having a releasable coupler mounted thereon; and a mandrel having a distal end configured to form a prosthesis shell when coated with a polymer dispersion, and also having a proximal end configured to be received and engaged by the releasable coupler to hold the mandrel on the spindle. In one alternative aspect, the cassette has a plurality of spindles. In another aspect, the proximal end of the mandrel has a hexagonal shape. In still another aspect, the releasable coupler is a quick connect device. In yet another aspect, the cassette further includes a drive assembly for driving the rotation of the spindle.

In still another aspect, the cassette further comprises a battery and a motor powered by the battery, the motor configured to drive the spindle. In one alternative aspect, the drive assembly is configured to be driven by a drive motor that is configured to engage the drive assembly but which is separate from the cassette. In another alternative aspect, the cassette further comprises electrical contacts disposed on an end of the cassette and configured to receive electrical power from a power supply external to the cassette, the cassette also having a motor in electrical communication with the electrical contacts, the motor configured to drive the spindle.

In another aspect, the present invention includes an automated process for manufacturing prosthesis shells, comprising: loading a mandrel configured to form a prosthesis shell into a cassette, positioning the loaded cassette at a dipping station using a robot controlled by a controller having a processor and memory for storing programming commands for controlling operation of the controller and also for storing information related to manufacturing of the prosthesis shells; pumping a first dispersion formed from a polymer and a solvent, under control of the controller, to lay down a base layer on the mandrel; positioning the loaded cassette at a devolatilization station using the robot to evaporate at least a portion of the solvent from the layer of polymer dispersion on the mandrel; positioning the loaded cassette at a barrier station using the robot; pumping a second dispersion formed from a barrier material and a solvent, under control of the controller, to lay down a barrier layer on the mandrel; and positioning the loaded cassette, using the robot, in a curing oven to cure the base layer and the barrier layer. In yet another aspect, multiple base layers may be applied to the mandrel by repeatedly positioning, using the robot, the loaded cassette at the dipping station and devolatilization stations.

In another aspect, the controller controls a driver engaged with the mandrel to rotate the mandrel when the first dispersion is being pumped onto the mandrel. In an alternative aspect, the controller controls a driver engaged with the mandrel to rotate the mandrel when the second dispersion is being pumped onto the mandrel. In still another alternative aspect, the cassette includes a battery that powers a driver engaged with the mandrel to rotate the mandrel.

In yet another aspect, the dipping station, the devolatilization station, and the barrier station are located within an enclosed area to provide for control of solvent vapor emissions. In another aspect, the process further comprises removing the solvent vapor emissions from the enclosed area and processing the solvent vapor emissions resulting in a post-processing solvent vapor emission level that is less than a pre-processing solvent vapor emission level. In one alternative aspect, the post-processing solvent vapor emission is at least ninety percent less than the pre-processing solvent vapor emission level.

Other features and advantages of the invention will become apparent from the following detailed description, taken in conjunction with the accompanying drawings, which illustrate, by way of example, the features of the invention.

### BRIEF DESCRIPTION OF THE DRAWINGS

FIG. 1 is a perspective top view of one embodiment of a mandrel in accordance with principles of the present invention.
FIG. 2 is an enlarged view of a proximal end of the mandrel of FIG. 1.
FIG. 3 is a partially cut-away perspective view of an embodiment of a cassette, showing mandrels releasably mounted to the cassette and details of one embodiment of a drive system used to rotate the mandrels.
FIG. 4 is a graphical representation of an automated manufacture process used to manufacture prosthesis shells, showing mandrel loaded onto cassettes in a cassette loading station.
FIG. 5 is a graphical representation of an automated manufacture process used to manufacture prosthesis shells, showing a loaded cassette positioned at a dipping station by a robot.
FIG. 6 is a graphical representation of an automated manufacture process used to manufacture prosthesis shells, showing loaded cassettes placed into a devolatilization station after a base layer has been laid down on each mandrel mounted onto the cassette.
FIG. 7 is a graphical representation of an automated manufacture process used to manufacture prosthesis shells, showing a loaded cassette positioned at a barrier dipping station by the robot.
FIG. 8 is a graphical representation of an automated manufacture process used to manufacture prosthesis shells, showing loaded cassettes positioned in a curing oven.
FIG. 9 is a graphical representation of an embodiment of a control system that may be used to control the operation of an automated prosthesis shell manufacturing process.

### DETAILED DESCRIPTION OF THE PREFERRED EMBODIMENTS

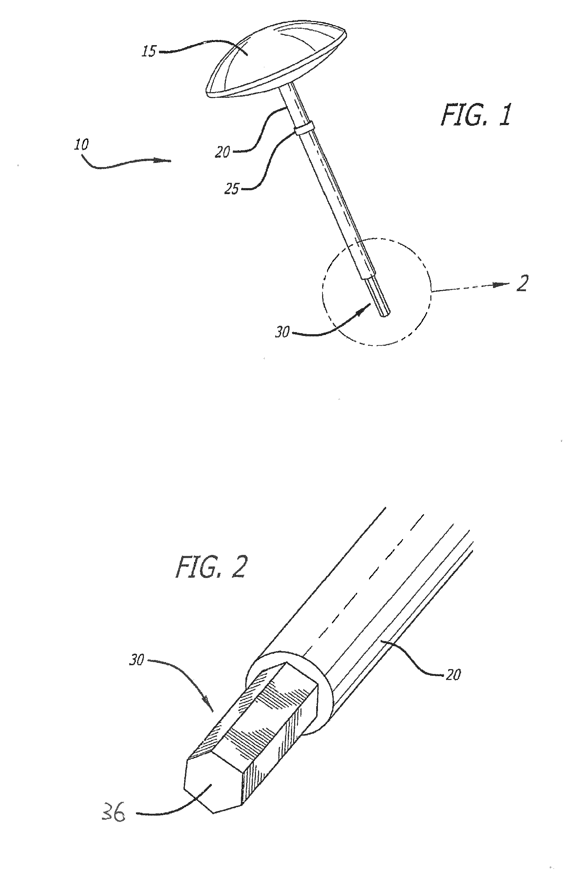

Referring now to the drawings in detail, in which like reference numerals indicate like or corresponding elements among the several figures, there is shown in FIG. 1 an exemplary embodiment of a mandrel 10 in accordance with the present invention. Mandrel 10 has a molding surface 15 disposed at a distal end of the mandrel. The molding surface 15 may be shaped such that a shell that is molded on the molding surface will have a desired shape, such as, for example, but not limited to, an anatomically correct shape designed to mimic a human breast. The various specifications of the molding surface, such as the width, height and depth of the molding surface are selected depending on the size, shape or other characteristic of the desired resultant molded shell.

A bottom side of the molding surface 15 is mounted, typically removably, but not necessarily, to a distal end of a rod 20. Rod 20 may be round, or may have another shape. Rod 20 may also be formed by connecting various lengths of rods using a coupler 25. In this manner, the length of rod 20 may be varied as needed. A proximal end 30 of rod 20 may be shaped differently than the remainder of rod 20 so as to facilitate mounting of rod 20 in a mounting device, such as a releasable chuck.

FIG. 2 illustrates one example of how proximal end 30 may be shaped to facilitate mounting mandrel 10 to a fixture or other mounting device to allow mandrel 10 to be rotated and transported during manufacture of a shell. In this exemplary embodiment, proximal end 30 has a hexagonal shaped portion 36, which may, as shown, have a smaller diameter than the remainder of rod 20. Alternatively, the diameter of the hexagonal shaped portion 36 of proximal end 30 may have the same, or larger, diameter as rod 20. Those skilled in the art will immediately understand that while a hexagonal portion is shown, other shapes, such as octagonal, square or other shapes may be used and are contemplated to be within the scope of the invention.

In another embodiment, proximal end 30 is configured in accordance with commonly known "quick release" fittings so that it may be releasably mounted to a "quick release" chuck or other mounting device.

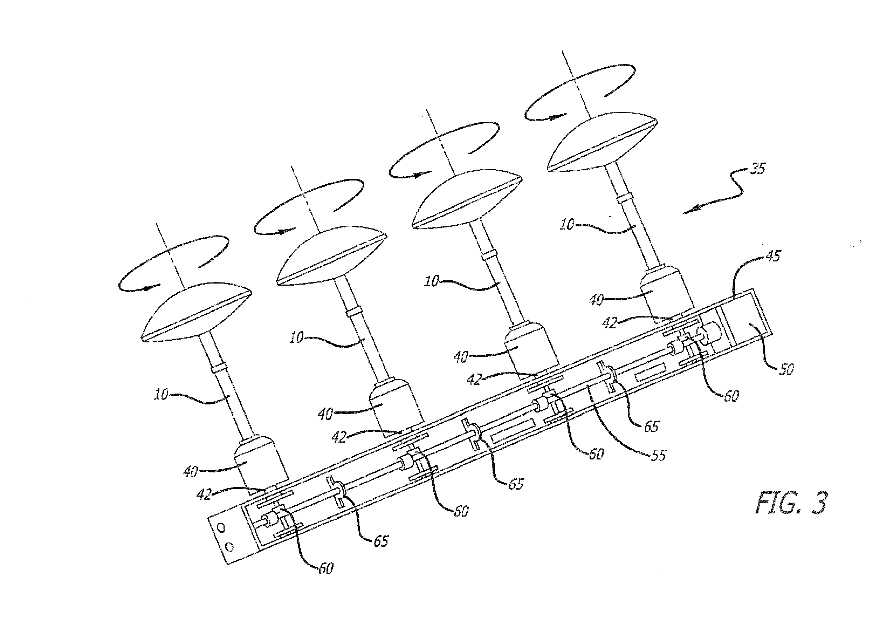

FIG. 3 illustrates one embodiment of a modular cassette 35 capable of being loaded with a plurality of mandrels 10. In this embodiment, mandrels 10 are removably loaded into mounting devices 40. Each mounting device 40 is mounted on a shaft 42 which extends from housing 45. As one skilled in the art understands, shaft 42 may be mounted on bearings or other friction reducing device that are disposed in holes in two opposite sides of housing 45 so as to both support shaft 42 and to allow shaft 42 to rotate freely so as to drive rotation of mounting device 40 and thus mandrels 10.

A motor 50 may be mounted at one end of housing 45. Motor 50 may drive a shaft 55 that extends along the length of housing 45. Shaft 55 may be supported within housing 45 by one or more bearing/support assemblies 65. Shaft 55 is configured to engage each of shafts 42 in a manner that allows rotation of shaft 55 to be transmitted to shaft 42 so as to drive the rotation of the mandrels 10. Such engagement may take a variety of forms, including, but not limited to, friction transmission, gear and cog transmission and the like. Motor 50 may also include appropriate gearing to achieve a desired rate of rotation.

Alternatively, motor 50 may be a variable speed motor. In one embodiment, the motor is powered by a battery (not shown) that resides in the cassette. This battery will typically be rechargeable, such as a NiCd or Lithium based battery. However, the battery may also be a non-rechargeable battery. In these embodiments, the cassette may be considered to be "cordless" in that the cassette may be operated without being tethered to a power supply.

In still another embodiment, the motor may be powered by a power supply that is external to the cassette. For example, but without limitation, as will be discussed in more detail below, the cassette may be picked up by a robotic arm and positioned at various stations used to manufacture prosthesis shells. In one embodiment, the robotic arm may be configured to supply power to the motor through electrical contacts on the arm that engage similar electrical contacts mounted on the cassette when the cassette is picked up by the robotic arm.

In yet another embodiment, motor 50 may be replaced by a drive assembly that is configured to engage a motor that is not part of cassette 35. In this embodiment, the cassette may be removably mounted to a structure that includes a motor in such a way that the motor engages the drive assembly of the cassette when the cassette is mounted on the structure.

In another embodiment, each of the mandrels mounted to the cassette may be driven by a separate motor. In this manner, the speed of rotation of each mandrel may be individually controlled.

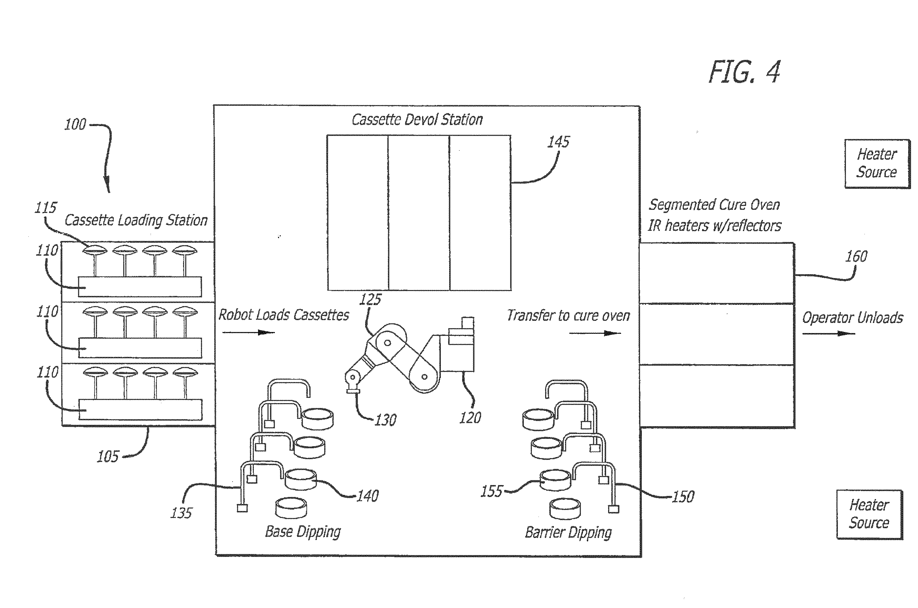

FIG. 4 illustrates one embodiment of an automated system for manufacturing prosthesis shells in accordance with various principles of the present invention. In the illustrated embodiment, mandrels 115 are loaded onto cassettes 110. While this illustration shows cassettes as being capable of holding four mandrels, other configurations are possible. The loaded cassettes are placed into a cassette loading station 105.

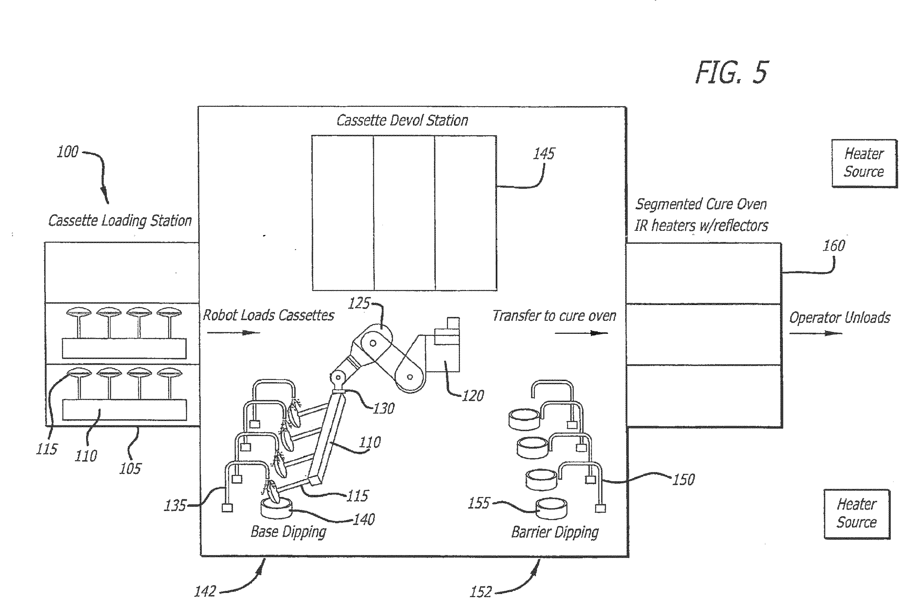

FIG. 5 illustrates the next step employed by the automated system to manufacture prosthesis shells. A computer controlled robot 120 having an articulating ann 125 and configured to move in six axes extends and picks up a loaded cassette 110 from cassette loading station 105. Articulating arm 125 has a coupler 130 configured to engage an end of the loaded cassette 110. Coupler 130 may include a motor drive (not shown) that engages a drive assembly disposed in the end of the cassette that then provides rotational movement to the drive assembly of the cassette so as to rotate the mandrels 115.

Once the robot 120 has picked up the loaded cassette, the articulating arm of the robot is commanded by software commands executing on the computer that controls the robot to position the loaded cassette under nozzles 135 of a base dipping station. Once positioned under nozzles 135, a controller, which may be the same computer controlling the robot, or it may be different controller that is programmed to carry out various steps of the process and is in communication with the computer controlling the robot, commands a pump (not shown) to pump a stream of liquid silicone dispersed in a solvent through nozzles 135 onto the rotating mandrels 115 for a selected period of time. The speed of rotation of the mandrels and the duration of time that the stream of silicone is directed at the mandrels is controlled by the controller to ensure that a uniform coating of liquid silicone is applied to the mandrels.

After a period of time, the pump is shut off, stopping the stream of liquid silicone flowing from nozzles 135. The cassette may be held in position for another period of time to allow excess silicone to drain from the mandrel. During this time, the mandrel may continue to rotate at the same speed as during application of the silicone, or the speed may be varied to obtain desired characteristics of the coating. As shown, the excess silicone may be drained into drains 140, and may be recycled for further use.

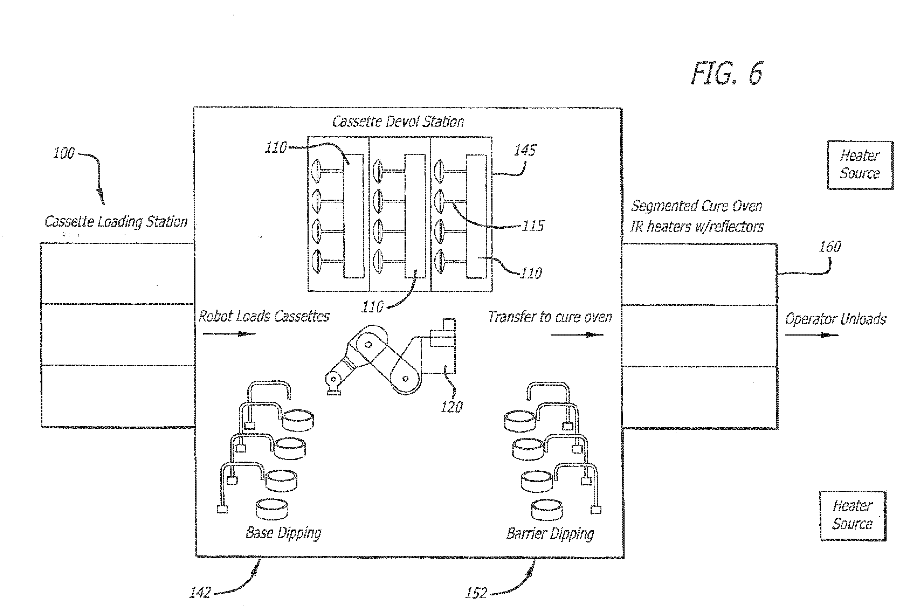

After excess silicone has been drained from the mandrels 115 mounted on the cassette 110, the robot 120 is controlled to place the loaded cassette into a cassette devolatilization station 145 shown in FIG. 6. The loaded cassette remains in the devolatilization station 145 for a predetermined period of time, such as, for example 5 to 60 minutes, and preferably 10 minutes, until most, if not all, of the solvent mixed with the silicone is evaporated from the silicone coating disposed on the mandrels.

While FIG 6. illustrates the devolatilization station 145 as holding three loaded cassettes, a skilled person will understand that the station may be configured to hold more or fewer than three loaded cassettes, and that only a single loaded cassette may be placed in the devolatilization station without departing from the intended scope of the invention. It will also be understood that the controller may keep track of the time a loaded cassette is placed into the devolatilization station and control the robot to remove an individual loaded cassette after a specified period of time, leaving the remaining cassettes in the devolatilization station. In this manner the automated system may ensure that cassettes are continuously moving through the system in an ordered process that ensures optimum efficiency.

After a cassette is devolatilized, the controller may control robot 120 to pick up the devolatilized cassette from the devolatilization station 145 and once again position the devolatilized cassette in the base dipping station 142 as shown in FIG. 5 to add another layer of silicone on top of the devolatilized layer of silicone. After this layer has been laid down, the robot will again place the coated mandrels and cassette in the devolatilization station 145 for devolatilization of the second layer of silicone. This process may be repeated as many times as necessary to build up a shell having a desired thickness.

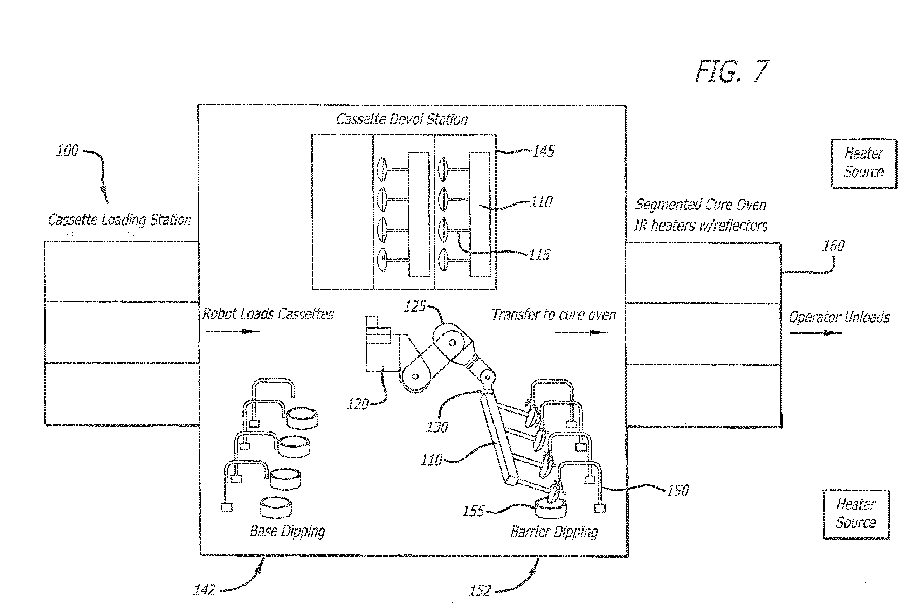

FIG. 7 illustrates another embodiment of the automated system wherein a barrier layer is applied to the mandrels of a cassette. In this embodiment, robot 120 is controlled to pick up a loaded, devolatilized cassette 110 from devolatilization station 145 and position the loaded devolatilized cassette in a barrier dipping station 152. The barrier material, which may be a silicone dispersed in a solvent having desired properties is streamed onto the mandrels 115 through nozzles 150. Excess barrier material is allowed to drain into drains 155. In some embodiments, the loaded cassettes having mandrels that include a barrier layer may be placed back into the devolatilization station 145 to devolatilize the barrier layer if necessary.

Depending on the process desired, the robot picks up the loaded cassette 110 having mandrels which have been coated with a desired thickness of silicone, and which may also include a barrier layer, from the devolatilization station 145 and places the loaded cassette into a curing oven 160. Curing oven may include heat sources 165 to assist in curing the prosthesis shell to a desired level of polymerization and/or cross-linking of the various layers so as to provide a shell having a uniform thickness and desirable properties such as feel, modulus and hardness. The heat sources may be hot air blowers, or they may be infrared heaters, or a combination of both.

After the loaded cassettes have been cured for a desired period of time, such as, for example, 20 to 180 minutes, and preferably 60 minutes, the loaded cassettes are removed from the cure oven for quality checks.

The finished shells may now be removed from the mandrels. Because the mandrels, as shown in FIG. 3 are removably mounted to the cassettes, they may be easily removed for further processing. Such processing includes removal of the shell from the mandrel, patching of the hole left in the shell by the rod of the mandrel, and filling of the patched shell with gel or saline to complete the prosthesis.

One skilled in the art will appreciate that the disclosed system for manufacturing shells is advantageous over prior systems in that it allows for efficient automation of the shell manufacturing process. The combination of ease in mounting cassettes, and the use of cassettes in a robotically manipulated process, allows for improved consistency and uniformity of shell formation. Automating the process also provides for reduced cost of production in that fewer operators are necessary due to use of the robot to move the cassettes through the process. Most prior systems relied on manual manipulation of a single mandrel by a human through the various stages of the process. Not only could fewer mandrels be processed in a given period of time, but positioning of the mandrel, and differences in times of exposing the mandrel to the flow of silicone and devolatilization by an operator often results in inconsistent and non-uniform shells.

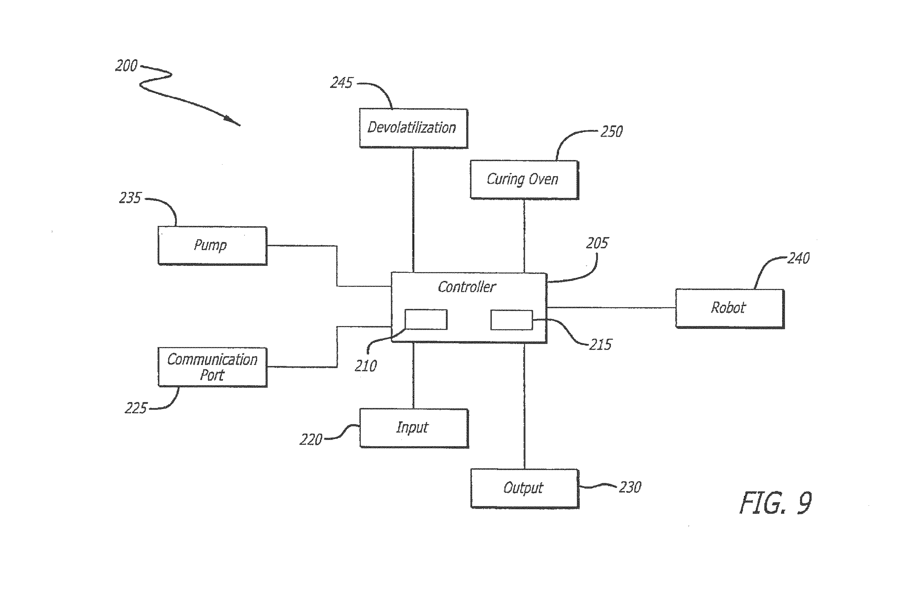

FIG. 9 illustrates one embodiment of a control system 200 that may be used to carry out and control the various processes of the system and method of the present invention. Control system 200 includes a controller 205 which may be tasked with controlling the overall operation of the system. Controller 205 will typically include a processor 210 and a memory 215. Memory 215 maybe configured to store software programs to be executed by processor 210, and may also be used to store information related to the operation of the system, including, but not limited to, operational parameters used by the operating software to control the system, records of manufacture that are related to the manufacture of individual or batches of shells, and other process related data that may be useful for monitoring and controlling the operation of the system.

Controller 205 may also be in operable communication with an input device, which may be any device known to those skilled in the art, such as, for example, but not limited to, a keyboard, mouse and the like. Controller 205 will also typically be provided with a communication port 225 which allows for communication between controller 205 and other systems or memories that may accessible to controller 205. For example, controller 205 may be in communication through communication port 225 to other computers, processor, servers, databases and the like through a local or wide-area wired or wireless network. Communication port 225 may also be configured to allow communication of programming commands to be executed by processor 210 to be uploaded to the memory 215.

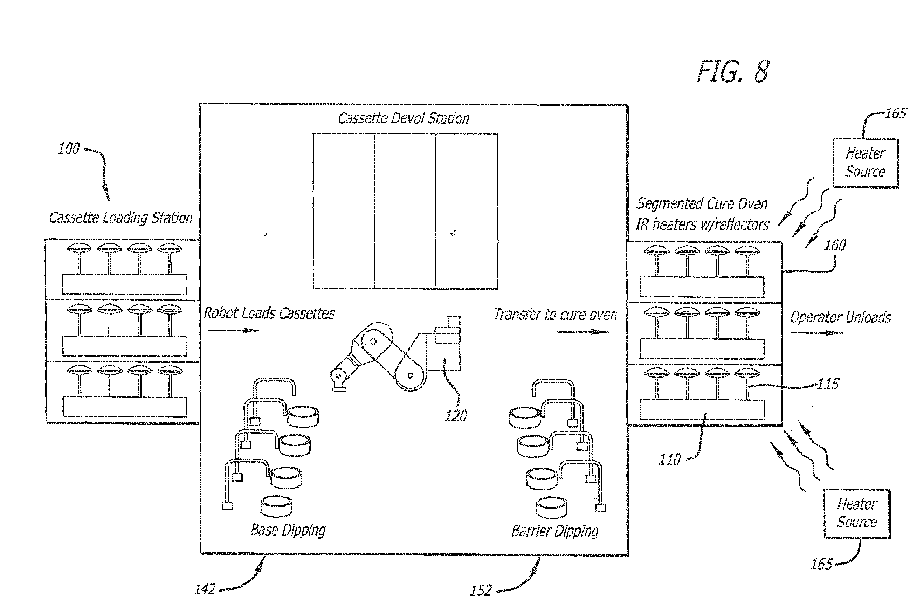

As described with reference to FIGS. 4-8 above, controller 205 may be programmed to control one or more pumps 235 to control the flow of silicone base and/or barrier fluid to coat the mandrels. Controller 205 may also be programmed to control the actions of robot 240, including manipulation and movement of the robot's arms, and the speed of rotation of the mandrels picked up by the robot arm and actuated by coupler 130 of articulating arm 125 (FIG. 4) of the robot. Controller 205 may also be programmed to control the operation of the devolatilization station 245, as well as to control the curing process using curing oven 25.

The various embodiments of the invention described above are advantageous in that using an untethered cassette allows the entire dipping and devolatilization process to be housed in a closed system that provides for improved solvent vapor control, ultra clean processing and controlled devolatilization with minimal air volume exchange. Using the system and methods of the various embodiments described above may eliminate the need for a large clean room utilizing single pass air exchange, the air handling costs of which are typically on the order of $50,000 per month or greater. Use of the embodiments of the present invention may reduce that cost by 90% or more.

Utilizing a closed system as described above reduces the foot print of the manufacturing process because use of the robot essentially eliminates the need for operators to be present during the dipping and devolatilization processes. Such a system also provides for extraction or removal of the solvent vapor from the closed system in a manner that allows the extracted vapor emissions to be channeled to a solvent recovery system. Such a solvent recovery system may be, for example, but not limited to, a vapor oxidation system or other type of solvent recovery system. In some embodiments, solvent vapor concentration after channeling the solvent vapor through the recovery system may be reduced by ninety percent or more from the original solvent vapor concentration.

While the various embodiments of the present invention have been discussed with reference to a mandrel suitable for use in a dipping or spraying process, those skilled in the art will appreciate that other types of molds can be used to form prosthesis shells utilizing the principles of the present invention. For example, but not limited to, a rotational mold may be mounted to the spindle of a cassette. In such a mold, polymer dispersion is poured or otherwise added to the mold, and the mold is rotated while air or other gas is directed at the mold to devolatilize the dispersion. In another non-limiting example, a two part mold may be mounted to the spindle. Polymer dispersion is inserted or added to the interior of the mold, a vacuum is applied to the mold, and the mold is rotated in multiple axes to ensure that the dispersion coats the interior surface of the two part mold to form the prosthesis shell.

While several particular forms of the invention have been illustrated and described, it will be apparent that various modifications can be made without departing from the scope of the invention as defined by the claims.

## Claims

1. A mounting system for mounting a mandrel configured to form a prosthesis shell to a cassette, comprising:
a cassette (35) having a spindle (42), the spindle rotatably mounted to the cassette, the spindle having a proximal end configured to be driven so as to impart rotation to the spindle, the spindle also having a distal end having a releasable coupler (130) mounted thereon; and
a mandrel (10) having a distal end configured to form a prosthesis shell when coated with a polymer dispersion, and also having a proximal end configured to be received and engaged by the releasable coupler (40) to hold the mandrel on the spindle.

2. The system of claim 1, wherein the cassette has a plurality of spindles.

3. The system of claim 1, wherein the proximal end of the mandrel has a hexagonal shape (36).

4. The system of claim 1, where the releasable coupler is a quick connect device.

5. The system of claim 1, wherein the cassette further includes a drive assembly for driving the rotation of the spindle.

6. The system of claim 1, wherein the cassette further comprises a battery and a motor (50) powered by the battery, the motor configured to drive the spindle.

7. The system of claim 1, wherein the drive assembly is configured to be driven by a drive motor that is configured to engage the drive assembly but which is separate from the cassette.

8. The system of claim 1, wherein the cassette further comprises electrical contacts disposed on an end of the cassette and configured to receive electrical power from a power supply external to the cassette, the cassette also having a motor in electrical communication with the electrical contacts, the motor configured to drive the spindle.

9. An automated process for manufacturing prosthesis shells, comprising:
loading a mandrel (115) configured to form a prosthesis shell into a cassette,
positioning the loaded cassette (110) at a dipping station (142) using a robot (120) controlled by a controller having a processor and memory for storing programming commands for controlling operation of the controller and also for storing information related to manufacturing of the prosthesis shells;
pumping a first dispersion formed from a polymer and a solvent, under control of the controller, to lay down a base layer on the mandrel;
positioning the loaded cassette at a devolatilization station (145) using the robot to evaporate at least a portion of the solvent from the layer of polymer dispersion on the mandrel;
positioning the loaded cassette at a barrier station (152) using the robot;
pumping a second dispersion formed from a barrier material and a solvent, under control of the controller, to lay down a barrier layer on the mandrel; and
positioning the loaded cassette, using the robot, in a curing oven (160) to cure the base layer and the barrier layer.

10. The process of claim 9, wherein multiple base layers may be applied to the mandrel by repeatedly positioning, using the robot, the loaded cassette at the dipping station and devolatilization stations.

11. The process of claim 9, wherein the controller controls a driver engaged with the mandrel to rotate the mandrel when the first dispersion is being pumped onto the mandrel, or wherein the controller controls a driver engaged with the mandrel to rotate the mandrel when the second dispersion is being pumped onto the mandrel.

12. The process of claim 9, wherein the cassette includes a battery that powers a driver engaged with the mandrel to rotate the mandrel.

13. The process of claim 9, wherein the dipping station (142), the devolatilization station (145), and the barrier station (152) are located within an enclosed area to provide for control of solvent vapor emissions.

14. The process of claim 13, further comprising removing the solvent vapor emissions from the enclosed area and processing the solvent vapor emissions resulting in a post-processing solvent vapor emission level that is less than a pre-processing solvent vapor emission level.

15. The process of claim 14, wherein the post-processing solvent vapor emission is at least ninety percent less than the pre-processing solvent vapor emission level.
